(19)

**Europäisches Patentamt**
**European Patent Office**
**Office européen des brevets**

(11) **EP 4 151 998 B1**

(12) **EUROPEAN PATENT SPECIFICATION**

(45) Date of publication and mention
of the grant of the patent:
**17.09.2025 Bulletin 2025/38**

(51) International Patent Classification (IPC):
**G01N 21/64** *(2006.01)*

(21) Application number: **21803025.2**

(52) Cooperative Patent Classification (CPC):
**G01N 21/6456**

(22) Date of filing: **27.04.2021**

(86) International application number:
**PCT/KR2021/005322**

(87) International publication number:
**WO 2021/230535 (18.11.2021 Gazette 2021/46)**

(54) **METHOD FOR EVALUATING EFFICACY OF UV-BLOCKING INGREDIENT FOR HAIR**

VERFAHREN ZUR BEWERTUNG DER WIRKSAMKEIT EINES UV-BLOCKIERENDEN
WIRKSTOFFS FÜR HAARE

PROCÉDÉ PERMETTANT D'ÉVALUER L'EFFICACITÉ D'UN INGRÉDIENT BLOQUANT LES UV
POUR LES CHEVEUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.05.2020 KR 20200056015**

(43) Date of publication of application:
**22.03.2023 Bulletin 2023/12**

(73) Proprietor: **P&K Skin Research Center**
**Seoul 07236 (KR)**

(72) Inventors:
• **SHIN, Jin-Hee**
  **Seoul 06608 (KR)**
• **LEE, Hae-Kwang**
  **Suwon-si, Gyeonggi-do 16493 (KR)**
• **LEE, Jeong-Ok**
  **Gwangmyeong-si, Gyeonggi-do 14319 (KR)**
• **YANG, Nu-Ri**
  **Seoul 07776 (KR)**

(74) Representative: **Plasseraud IP**
**104 Rue de Richelieu**
**CS92104**
**75080 Paris Cedex 02 (FR)**

(56) References cited:
JP-A- H08 271 515    JP-A- H08 271 515
KR-A- 20060 127 405   US-A1- 2019 285 546

• HERRWERTH S ET AL: "Testing polysilicone-19 for hair conditioning and uv protection claims", COSMETICS & TOILETRIES MAGAZINE, ALLURED PUBLISHING CORP, US, vol. 123, no. 5, 1 May 2008 (2008-05-01), pages 101 - 110, XP009134523, ISSN: 0361-4387
• SANDHU S S ET AL: "A SENSITIVE FLUORESCENCE TECHNIQUE USING DANSYL CHLORIDE TO ASSESS HAIR DAMAGE", JOURNAL OF THE SOCIETY COSMETIC CHEMISTS, SOCIETY OF COSMETIC CHEMISTS, US, vol. 40, no. 5, 1 September 1989 (1989-09-01), pages 287 - 296, XP009144064, ISSN: 0037-9832
• YUEN C. W. M., KAN C. W., CHOW Y. L.: "Effect of sun protection agent on preventing hair colour fading and hair damage", FIBERS AND POLYMERS, vol. 11, no. 2, 1 April 2010 (2010-04-01), Seoul, pages 316 - 320, XP055866183, ISSN: 1229-9197, DOI: 10.1007/s12221-010-0316-1
• SANDHU S S , ROBINS C R: "A sensitive fluorescence technique using dansyl chloride to assess hair damage", JOURNAL OF THE SOCIETY COSMETIC CHEMIST, vol. 40, no. 5, 1 September 1989 (1989-09-01), pages 287 - 296, XP009144064, ISSN: 037-9832

EP 4 151 998 B1

- **CHENG S. Y., YUEN C. W. M., KAN C. W., CHEUK K. K. L: "Effect of Hair Damage on the Colour Uptake of an Oxidizing Semi-permanent Colorant", FIBERS AND POLYMERS, vol. 9, no. 3, 1 June 2008 (2008-06-01), Seoul, pages 341 - 348, XP055866189, ISSN: 1229-9197, DOI: 10.1007/ s12221-008-0055-8**

## Description

[Technical Field]

The present disclosure relates to a method for evaluating the efficacy of a UV-blocking ingredient for hair, specifically to a method for evaluating the efficacy of a UV-blocking ingredient by applying the UV-blocking ingredient to a hair sample treated with a fluorescent dye and measuring the difference in chromaticity before and after UV irradiation. This application claims the priority of Korean Patent Application No. 10-2020-0056015, filed on May 11, 2020.

[Background Art]

Hair is made up of the cuticle, the cortex and the medulla and the outermost layer is the cuticle. The cuticle is a thin layer that covers the outermost layer of hair and it is overlapped in a shape of scales. It protects hair from outside and affects the gloss of hair. Being composed of hard keratin proteins, it is brittle and vulnerable to friction. Therefore, it can be damaged or exfoliated easily by excessive combing or shampooing. The cortex is located inside of the cuticle and accounts for 80-90% of hair. It contains a lot of melanin pigment and is easily damaged by chemicals such as perming agents, hair dyes, etc. The medulla is located at the center of hair. A small number of melanin pigment particles exist in the medulla, and the medulla have many air pockets of various sizes, which contain air and absorb water. Damaged hair exhibits porosity. Porous hair absorbs more water but loses more moisture, making it easy to dry. Accordingly, whereas it is easy to produce strong waves by the perm agent for normal hair with a higher proportion of the medulla, it is difficult and takes longer to produce waves for damaged hair with a lower proportion of the medulla.
The thickness of the cuticle is decreased when UV is irradiated to hair, which is easily damaged by the external environment. The UV irradiation leads to the destruction of cells, cell membrane complexes and melanin, and the porosis of the cortex occurs due to the exfoliation of the cuticle, resulting in splitting or cutting of hair.
Although Korean Patent Publication No. 2006-0127405 discloses a method of evaluating hair damage by specifically fluorescence-labeling the carbonyl group of oxidized proteins in hair, a method for evaluating the efficacy of a UV-blocking ingredient for protecting hair from UV is not known widely.
"Testing polysilicone-19 for hair conditioning and uv protection claims", Cosmetic&Toiletries magazine, allured publishing corp (vol. 123, no. 5, 1 May 2008, JP H08 271515 A, "A sensitive fluorescence technique using dansyl chloride to assess hair damage", Journal of the society cosmetic chemists, society of cosmetic chemists (vol. 40, no. 5, 1 September 1989), and US 2019/285546 A1 disclose a method for evaluating hair damage.

[References of Related Art]

[Patent Documents]

(Patent document 1) Korean Patent Publication No. 2006-0127405.

**[Disclosure]**

[Technical Problem]

The inventors of the present disclosure have made efforts to provide a method for evaluating the efficacy of a UV-blocking ingredient by treating a hair sample with a fluorescent dye. As a result, they have found out that the efficacy of a UV-blocking ingredient can be evaluated by applying the UV-blocking ingredient to a hair sample treated with a fluorescent dye or applying the UV-blocking ingredient onto a part of a UV-permeable plate and then calculating a UV cut-off rate based on the difference in chromaticity before and after UV irradiation, and have completed the present disclosure.
The present disclosure is directed to providing a method for evaluating the efficacy of a UV-blocking ingredient by treating a hair sample with a fluorescent dye.

[Technical Solution]

The present disclosure provides a method for evaluating the efficacy of a UV-blocking ingredient as defined in the appended set of claims, the method includes:

a step of treating a hair sample with a fluorescent dye;
a step of applying a UV-blocking ingredient to the hair sample treated with the fluorescent dye;
a step of measuring chromaticity by fluorescence-imaging the hair sample with the UV-blocking ingredient applied and a hair sample with the UV-blocking ingredient not applied;

a step of irradiating UV to the hair samples;

a step of measuring chromaticity by fluorescence-imaging the hair samples; and

a step of calculating a UV cut-off rate by comparing the difference in chromaticity ($\Delta E_1$) or difference in brightness ($\Delta L_1$) of the hair sample with the UV-blocking ingredient applied before and after UV irradiation with the difference in chromaticity ($\Delta E_2$) or difference in brightness ($\Delta L_2$) of the hair sample with the UV-blocking ingredient not applied before and after UV irradiation.

The fluorescent dye may be dansyl chloride.

The hair sample may be immersed in an organic solvent in which the dansyl chloride is dissolved for 0.5-48 hours.

The UV may be irradiated with an intensity of 1,000-100,000 mJ/cm$^2$.

The chromaticity may be a measured value according to CIE LAB.

The difference in chromaticity ($\Delta E_1$) of the hair sample with the UV-blocking ingredient applied before and after UV irradiation may be calculated according to Equation 1, and the difference in chromaticity ($\Delta E_2$) of the hair sample with the UV-blocking ingredient not applied may be calculated according to Equation 2.

[Equation 1]

$$\Delta E_1 = ((L_2^* - L_1^*)^2 + (a_2^* - a_1^*)^2 + (b_2^* - b_1^*)^2)^{1/2}$$

In Equation 1,

$L_2^*$ is the brightness of the hair sample with the UV-blocking ingredient applied after UV irradiation, and $L_1^*$ is the brightness of the hair sample with the UV-blocking ingredient applied before UV irradiation,

$a_2^*$ is the chroma of the hair sample with the UV-blocking ingredient applied after UV irradiation, and $a_1^*$ is the chroma of the hair sample with the UV-blocking ingredient applied before UV irradiation, and

$b_2^*$ is the yellowness of the hair sample with the UV-blocking ingredient applied after UV irradiation, and $b_1^*$ is the yellowness of the hair sample with the UV-blocking ingredient applied before UV irradiation.

[Equation 2]

$$\Delta E_2 = ((L_4^* - L_3^*)^2 + (a_4^* - a_3^*)^2 + (b_4^* - b_3^*)^2)^{1/2}$$

In Equation 2,

$L_4^*$ is the brightness of the hair sample with the UV-blocking ingredient not applied after UV irradiation, and $L_3^*$ is the brightness of the hair sample with the UV-blocking ingredient not applied before UV irradiation,

$a_4^*$ is the chroma of the hair sample with the UV-blocking ingredient not applied after UV irradiation, and $a_3^*$ is the chroma of the hair sample with the UV-blocking ingredient not applied before UV irradiation, and

$b_4^*$ is the yellowness of the hair sample with the UV-blocking ingredient not applied after UV irradiation, and $b_3^*$ is the yellowness of the hair sample with the UV-blocking ingredient not applied before UV irradiation.

The UV cut-off rate may be calculated according to Equation 3.

[Equation 3]

UV cut-off rate (%) = (($\Delta E_2$ - $\Delta E_1$) / $\Delta E_2$) × 100

The difference in brightness ($\Delta L_1$) of the hair sample with the UV-blocking ingredient applied before and after UV irradiation may be calculated according to Equation 4, and the difference in brightness ($\Delta L_2$) of the hair sample with the UV-blocking ingredient not applied may be calculated according to Equation 5.

[Equation 4]

$$\Delta L_1 = |\, L_2^* - L_1^* \,|$$

[Equation 5]

$$\Delta L_2 = |\, L_4^* - L_3^* \,|$$

The UV cut-off rate may be calculated according to Equation 6.

[Equation 6]

$$\text{UV cut-off rate (\%)} = ((\Delta L_2 - \Delta L_1) / \Delta L_2) \times 100$$

The present invention provides a method for evaluating the efficacy of a UV-blocking ingredient as defined in the appended set of claims, the method includes:

a step of treating a hair sample with a fluorescent dye;
a step of measuring chromaticity by fluorescence-imaging the hair sample treated with the fluorescent dye;
a step of applying a UV-blocking ingredient to onto a part of a UV-permeable plate;
a step of locating the hair sample treated with the fluorescent dye below the UV-permeable plate;
a step of irradiating UV to the hair sample by irradiating UV from above the UV-permeable plate;
a step of measuring chromaticity by fluorescence-imaging the hair sample; and
a step of calculating a UV cut-off rate by comparing the difference in chromaticity ($\Delta E_3$) or difference in brightness ($\Delta L_3$) of the hair sample located below the part with the UV-blocking ingredient applied before and after UV irradiation with the difference in chromaticity ($\Delta E_4$) or difference in brightness ($\Delta L_4$) of the hair sample below the part with the UV-blocking ingredient not applied before and after UV irradiation.

[Advantageous Effects]

According to the present disclosure, the efficacy of a UV-blocking ingredient can be evaluated quantitatively by utilizing the difference in chromaticity of a hair sample before and after UV irradiation on the basis of the fact that, when UV is irradiated to the hair sample treated with a fluorescent dye, fluorescence intensity is decreased due to protein damage.

[Brief Description of Drawings]

FIG. 1a shows the fluorescence scanning microscopic image of a hair sample before UV irradiation.
FIG. 1b shows the fluorescence scanning microscopic image of a hair sample after UV irradiation.
FIG. 2 shows the images of a hair sample with a UV-blocking ingredient applied before and after UV irradiation.
FIG. 3 shows the images of a hair sample with a UV-blocking ingredient not applied before and after UV irradiation.

[Best Mode]

Hereinafter, the present disclosure is described in detail. Prior to description, the terms or words used in the present specification and claims should not be construed as being limited as commonly used or lexical meanings, and should be construed as meanings and concepts to conform with the technical idea disclosed in the present disclosure based on the principle that an inventor can appropriately define the concepts of terms to explain his/her invention in the best way. Accordingly, it should be understood that the exemplary embodiments described in the present specification are merely specific exemplary embodiments of the present disclosure and there can be various equivalents and modifications that can replace them at the time of this application.

The present disclosure provides a method for evaluating the efficacy of a UV-blocking ingredient, which includes:

a step of treating a hair sample with a fluorescent dye;
a step of applying a UV-blocking ingredient to the hair sample treated with the fluorescent dye;

a step of measuring chromaticity by fluorescence-imaging the hair sample with the UV-blocking ingredient applied and a hair sample with the UV-blocking ingredient not applied;

a step of irradiating UV to the hair samples;

a step of measuring chromaticity by fluorescence-imaging the hair samples; and

a step of calculating a UV cut-off rate by comparing the difference in chromaticity ($\Delta E_1$) or difference in brightness ($\Delta L_1$) of the hair sample with the UV-blocking ingredient applied before and after UV irradiation with the difference in chromaticity ($\Delta E_2$) or difference in brightness ($\Delta L_2$) of the hair sample with the UV-blocking ingredient not applied before and after UV irradiation.

First, a hair sample is treated with a fluorescent dye.

The hair sample may be gray hair, black hair, etc.

The fluorescent dye may be dansyl chloride.

The hair sample may be immersed in an organic solvent in which the dansyl chloride is dissolved for 0.5-48 hours, specifically for 0.5-10 hours, more specifically for 2-8 hours, most specifically for 3-5 hours. The organic solvent may be acetone. If the immersion time is shorter than the above-described range, fluorescence dyeing may be insufficient. However, if the immersion time is longer than the above-described range, the hair sample may be dissolved.

Then, a UV-blocking ingredient is applied to the hair sample treated with the fluorescent dye.

A hair sample with the UV-blocking ingredient applied and a hair sample with the UV-blocking ingredient not applied may be prepared separately, or a part of one hair sample may be applied with the UV-blocking ingredient and the remaining part may be not applied with the UV-blocking ingredient.

Chromaticity is measured by fluorescence-imaging the hair samples with the UV-blocking ingredient applied and not applied.

The chromaticity may be a measure value according to CIE LAB. Specifically, brightness (L*), chroma (a*) and yellowness (b*) values may be measured.

Then, UV is irradiated to the hair sample.

The UV may be irradiated with an intensity of 1,000-100,000 mJ/cm$^2$, specifically 2,000-50,000 mJ/cm$^2$, more specifically 3,000-30,000 mJ/cm$^2$, most specifically 5,000-10,000 mJ/cm$^2$. Specifically, the UV may be irradiated at 500-1,000 uw/cm$^2$ accumulated for 1-5 hours.

After the UV irradiation, chromaticity is measured again by fluorescence-imaging the hair sample.

A UV cut-off rate is calculated by comparing the difference in chromaticity ($\Delta E_1$) or difference in brightness ($\Delta L_1$) of the hair sample with the UV-blocking ingredient applied before and after UV irradiation with the difference in chromaticity ($\Delta E_2$) or difference in brightness ($\Delta L_2$) of the hair sample with the UV-blocking ingredient not applied before and after UV irradiation.

Specifically, the difference in chromaticity ($\Delta E_1$) of the hair sample with the UV-blocking ingredient applied before and after UV irradiation may be calculated according to Equation 1, and the difference in chromaticity ($\Delta E_2$) of the hair sample with the UV-blocking ingredient not applied may be calculated according to Equation 2.

[Equation 1]

$$\Delta E_1 = ((L_2^* - L_1^*)^2 + (a_2^* - a_1^*)^2 + (b_2^* - b_1^*)^2)^{1/2}$$

In Equation 1,

$L_2^*$ is the brightness of the hair sample with the UV-blocking ingredient applied after UV irradiation, and $L_1^*$ is the brightness of the hair sample with the UV-blocking ingredient applied before UV irradiation,

$a_2^*$ is the chroma of the hair sample with the UV-blocking ingredient applied after UV irradiation, and $a_1^*$ is the chroma of the hair sample with the UV-blocking ingredient applied before UV irradiation, and

$b_2^*$ is the yellowness of the hair sample with the UV-blocking ingredient applied after UV irradiation, and $b_1^*$ is the yellowness of the hair sample with the UV-blocking ingredient applied before UV irradiation.

[Equation 2]

$$\Delta E_2 = ((L_4^* - L_3^*)^2 + (a_4^* - a_3^*)^2 + (b_4^* - b_3^*)^2)^{1/2}$$

In Equation 2,

$L_4^*$ is the brightness of the hair sample with the UV-blocking ingredient not applied after UV irradiation, and $L_3^*$ is the brightness of the hair sample with the UV-blocking ingredient not applied before UV irradiation,

$a_4^*$ is the chroma of the hair sample with the UV-blocking ingredient not applied after UV irradiation, and $a_3^*$ is the chroma of the hair sample with the UV-blocking ingredient not applied before UV irradiation, and

$b_4^*$ is the yellowness of the hair sample with the UV-blocking ingredient not applied after UV irradiation, and $b_3^*$ is the yellowness of the hair sample with the UV-blocking ingredient not applied before UV irradiation.

The UV cut-off rate may be calculated according to Equation 3.

[Equation 3]

$$\text{UV cut-off rate (\%)} = ((\Delta E_2 - \Delta E_1) / \Delta E_2) \times 100$$

In addition, the difference in brightness ($\Delta L_1$) of the hair sample with the UV-blocking ingredient applied before and after UV irradiation may be calculated according to Equation 4, and the difference in brightness ($\Delta L_2$) of the hair sample with the UV-blocking ingredient not applied may be calculated according to Equation 5.

[Equation 4]

$$\Delta L_1 = | L_2^* - L_1^* |$$

[Equation 5]

$$\Delta L_2 = | L_4^* - L_3^* |$$

The UV cut-off rate may also be calculated according to Equation 6.

[Equation 6]

$$\text{UV cut-off rate (\%)} = ((\Delta L_2 - \Delta L_1) / \Delta L_2) \times 100$$

It may be determined that the UV-blocking ingredient has superior efficacy as the UV cut-off rate is higher.

The average value of the cut-off rates for an entire sample may be used by deriving UV-blocking index for a plurality of hair samples.

The present disclosure also provides a method for evaluating the efficacy of a UV-blocking ingredient, which includes:

a step of treating a hair sample with a fluorescent dye;
a step of measuring chromaticity by fluorescence-imaging the hair sample treated with the fluorescent dye;
a step of applying a UV-blocking ingredient to onto a part of a UV-permeable plate;
a step of locating the hair sample treated with the fluorescent dye below the UV-permeable plate;
a step of irradiating UV to the hair sample by irradiating UV from above the UV-permeable plate;
a step of measuring chromaticity by fluorescence-imaging the hair sample; and
a step of calculating a UV cut-off rate by comparing the difference in chromaticity ($\Delta E_3$) or difference in brightness ($\Delta L_3$) of the hair sample located below the part with the UV-blocking ingredient applied before and after UV irradiation with the difference in chromaticity ($\Delta E_4$) or difference in brightness ($\Delta L_4$) of the hair sample below the part with the UV-blocking ingredient not applied before and after UV irradiation.

The UV-permeable plate is a quartz plate, a band-pass filter, or PMMA (polymethyl methacrylate).

Through this, the efficacy of a UV-blocking ingredient may be evaluated by without applying the UV-blocking ingredient directly to a hair sample, applying the UV-blocking ingredient onto a part of the UV-permeable plate and then locating the hair sample below the UV-permeable plate. When the UV-blocking ingredient is applied directly to the hair sample, cleansing is required for the confirmation of chromaticity. However, the use of the UV-permeable plate allows the evaluation of efficacy without cleansing.

The conditions of the treatment with the fluorescent dye and the UV irradiation may be the same as those in the method of applying the UV-blocking ingredient directly to the hair sample.

The chromaticity may be a measured value according to CIE LAB. Specifically, brightness (L*), chroma (a*) and yellowness (b*) values may be measured.

The difference in chromaticity ($\Delta E_3$) of the hair sample located below the part with the UV-blocking ingredient applied before and after UV irradiation may be calculated according to Equation 7, and the difference in chromaticity ($\Delta E_4$) of the hair sample below the part with the UV-blocking ingredient not applied before and after UV irradiation may be calculated according to Equation 8.

[Equation 7]

$$\Delta E_3 = ((L_6^* - L_5^*)^2 + (a_6^* - a_5^*)^2 + (b_6^* - b_5^*)^2)^{1/2}$$

In Equation 7,

$L_6^*$ is the brightness of the hair sample located below the part with the UV-blocking ingredient applied after UV irradiation, and $L_5^*$ is the brightness of the hair sample located below the part with the UV-blocking ingredient applied before UV irradiation,

$a_6^*$ is the chroma of the hair sample located below the part with the UV-blocking ingredient applied after UV irradiation, and $a_5^*$ is the chroma of the hair sample located below the part with the UV-blocking ingredient applied before UV irradiation, and

$b_6^*$ is the yellowness of the hair sample located below the part with the UV-blocking ingredient applied after UV irradiation, and $b_5^*$ is the yellowness of the hair sample located below the part with the UV-blocking ingredient applied before UV irradiation.

[Equation 8]

$$\Delta E_4 = ((L_8^* - L_7^*)^2 + (a_8^* - a_7^*)^2 + (b_8^* - b_7^*)^2)^{1/2}$$

In Equation 8,

$L_8^*$ is the brightness of the hair sample located below the part with the UV-blocking ingredient not applied after UV irradiation, and $L_7^*$ is the brightness of the hair sample located below the part with the UV-blocking ingredient not applied before UV irradiation,

$a_8^*$ is the chroma of the hair sample located below the part with the UV-blocking ingredient not applied after UV irradiation, and $a_7^*$ is the chroma of the hair sample located below the part with the UV-blocking ingredient not applied before UV irradiation, and

$b_8^*$ is the yellowness of the hair sample located below the part with the UV-blocking ingredient not applied after UV irradiation, and $b_7^*$ is the yellowness of the hair sample located below the part with the UV-blocking ingredient not applied before UV irradiation.

The UV cut-off rate may be calculated according to Equation 9.

[Equation 9]

$$\text{UV cut-off rate (\%)} = ((\Delta E_4 - \Delta E_3) / \Delta E_4) \times 100$$

The difference in brightness ($\Delta L_3$) of the hair sample located below the part with the UV-blocking ingredient applied before and after UV irradiation may be calculated according to Equation 10, and the difference in brightness ($\Delta L_4$) of the hair sample located below the part with the UV-blocking ingredient not applied before and after UV irradiation may be calculated according to Equation 11.

[Equation 10]

$$\Delta L_3 = | L_6^* - L_5^* |$$

[Equation 11]

$$\Delta L_4 = | L_8{}^* - L_7{}^* |$$

The UV cut-off rate may be calculated according to Equation 12.

[Equation 12]

$$UV \text{ cut-off rate } (\%) = ((\Delta L_4 - \Delta L_3) / \Delta L_4) \times 100$$

The average value of the cut-off rates for an entire sample may be used by deriving UV-blocking index for a plurality of hair samples.

Hereinafter, the present disclosure will be described in detail through examples. However, the examples according to the present disclosure may be changed variously and it should not be construed that the scope of the present disclosure is limited by the examples. The examples of the present disclosure are provided to more fully describe the present disclosure to those having ordinary knowledge in the art.

**Example 1: Confirmation of damage of hair sample by UV irradiation**

First, a black hair sample was dyed by immersing in an acetone solution of 2 wt% dansyl chloride for 24 hours.

The fluorescence intensity of the dyed hair sample was measured using a UV lamp.

Then, after irradiating UV with a wavelength of 290-400 nm and an intensity of 800 uw/cm$^2$ to the hair sample for 3 hours, fluorescence intensity was measured again.

The fluorescence intensity was decreased from 193.57 before the UV irradiation to 154.66 after the UV irradiation by about 20%.

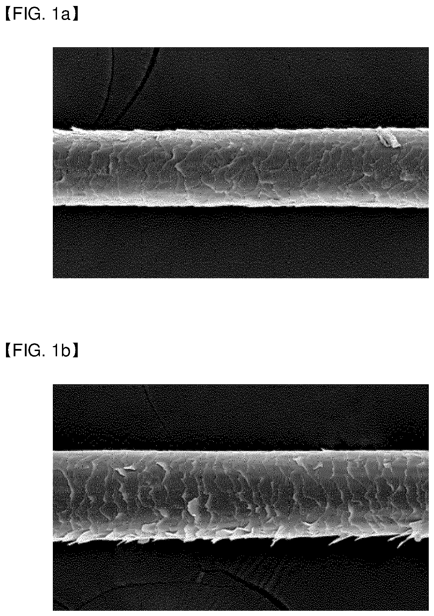

The fluorescence scanning microscopic images of the hair sample before and after the UV irradiation are shown in FIGS. 1a and 1b. As seen from FIGS. 1a and 1b, the cuticle was damaged significantly due to the UV irradiation.

**Example 2: Evaluation of efficacy of UV-blocking ingredient**

First, a black hair sample was dyed by immersing in an acetone solution of 2 wt% dansyl chloride for 30 minutes.

The chromaticity of the dyed hair sample was measured according to CIE LAB using a UV lamp (Visia CR mode).

Then, the UV-blocking ingredient(SPF 35) to be evaluated for efficacy was applied to the hair sample, and UV with a wavelength of 290-400 nm and an intensity of 800 uw/cm$^2$ was irradiated to the hair sample for 3 hours with a total dose of 8,640 mJ/cm$^2$.

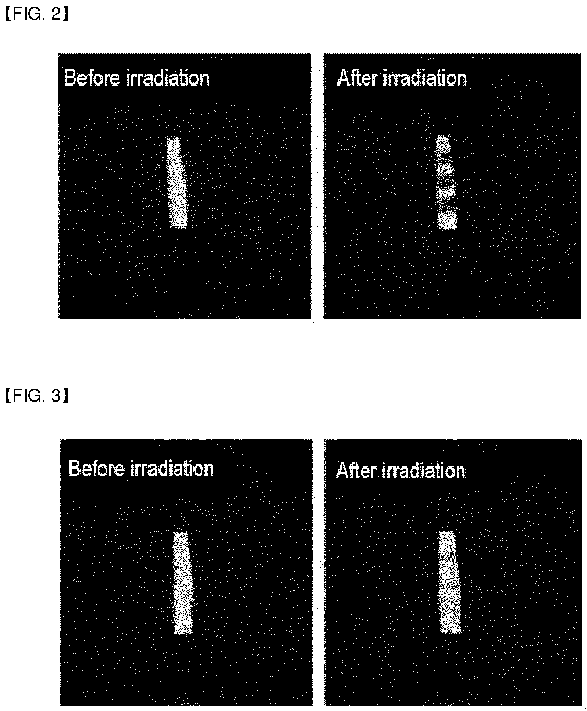

After the UV irradiation, the chromaticity of the hair sample with the UV-blocking ingredient applied was measured according to CIE LAB. The images of the hair sample before and after the UV irradiation are shown in FIG. 2.

The brightness, chroma and yellowness values according to CIE LAB before and after the UV irradiation are described in Table 1. The values are average values for three samples.

[Table 1]

| Before UV irradiation | | | After UV irradiation | | | Difference in chromaticity | | |
|---|---|---|---|---|---|---|---|---|
| L1* | a1* | b1* | L2* | a2* | b2* | ΔL | Δa | Δb |
| 29.231 | -3.972 | -26.112 | 15.489 | 2.607 | -24.459 | -13.742 | 6.579 | 1.653 |

In Table 1, $L_2{}^*$ is the brightness of the hair sample with the UV-blocking ingredient applied after the UV irradiation, $L_1{}^*$ is the brightness of the hair sample with the UV-blocking ingredient applied before the UV irradiation, $a_2{}^*$ is the chroma of the hair sample with the UV-blocking ingredient applied after the UV irradiation, $a_1{}^*$ is the chroma of the hair sample with the UV-blocking ingredient applied before the UV irradiation, $b_2{}^*$ is the yellowness of the hair sample with the UV-blocking ingredient applied after the UV irradiation, and $b_1{}^*$ is the yellowness of the hair sample with the UV-blocking ingredient applied before the UV irradiation.

$\Delta E_1$ was calculated as 15.33 according to Equation 1.

[Equation 1]

$$\Delta E_1 = ((L_2^* - L_1^*)^2 + (a_2^* - a_1^*)^2 + (b_2^* - b_1^*)^2)^{1/2}$$

Next, a black hair sample was dyed by immersing in an acetone solution of 2 wt% dansyl chloride for 30 minutes.

The chromaticity of the dyed hair sample was measured according to CIE LAB using a UV lamp (Visia CR mode).

Then, without applying a UV-blocking ingredient to the hair sample, UV with a wavelength of 290-400 nm and an intensity of 800 uw/cm² was irradiated to the hair sample for 3 hours with a total dose of 8,640 mJ/cm².

After the UV irradiation, the chromaticity of the hair sample was measured according to CIE LAB. The images of the hair sample before and after the UV irradiation are shown in FIG. 3.

The brightness, chroma and yellowness values according to CIE LAB before and after the UV irradiation are described in Table 2. The values are average values for three samples.

[Table 2]

| Before UV irradiation | | | After UV irradiation | | | Difference in chromaticity | | |
|---|---|---|---|---|---|---|---|---|
| L3* | a3* | b3* | L4* | a4* | b4* | ΔL | Δa | Δb |
| 27.75 | -1.517 | -27.852 | 12.066 | 4.446 | -23.11 | -15.684 | 5.963 | 4.742 |

In Table 2, $L_4^*$ is the brightness of the hair sample with the UV-blocking ingredient not applied after the UV irradiation, $L_3^*$ is the brightness of the hair sample with the UV-blocking ingredient not applied before the UV irradiation, $a_4^*$ is the chroma of the hair sample with the UV-blocking ingredient not applied after the UV irradiation, $a_3^*$ is the chroma of the hair sample with the UV-blocking ingredient not applied before the UV irradiation, $b_4^*$ is the yellowness of the hair sample with the UV-blocking ingredient not applied after the UV irradiation, and $b_3^*$ is the yellowness of the hair sample with the UV-blocking ingredient not applied before the UV irradiation.

$\Delta E_2$ was calculated as 17.44 according to Equation 2.

[Equation 2]

$$\Delta E_2 = ((L_4^* - L_3^*)^2 + (a_4^* - a_3^*)^2 + (b_4^* - b_3^*)^2)^{1/2}$$

The UV cut-off rate was calculated as 12.1% according to Equation 3.

[Equation 3]

$$\text{UV cut-off rate (\%)} = ((\Delta E_2 - \Delta E_1) / \Delta E_2) \times 100$$

In addition, the UV cut-off rate was calculated as 12.4% according to Equations 4-6.

[Equation 4]

$$\Delta L_1 = | L_2^* - L_1^* |$$

[Equation 5]

$$\Delta L_2 = | L_4^* - L_3^* |$$

[Equation 6]

$$\text{UV cut-off rate (\%)} = ((\Delta L_2 - \Delta L_1) / \Delta L_2) \times 100$$

The efficacy of the UV-blocking ingredient could be evaluated quantitatively by comparing the UV cut-off rate values.

**Claims**

1. A method for evaluating the efficacy of a UV-blocking ingredient, comprising:

   a step of treating a hair sample with a fluorescent dye;
   a step of measuring chromaticity by fluorescence-imaging the hair sample treated with the fluorescent dye;
   a step of applying a UV-blocking ingredient to onto a part of a UV-permeable plate;
   a step of locating the hair sample treated with the fluorescent dye below the UV-permeable plate;
   a step of irradiating UV to the hair samples by irradiating UV from above the UV-permeable plate;
   a step of measuring chromaticity by fluorescence-imaging the hair samples; and
   a step of calculating a UV cut-off rate by comparing the difference in chromaticity ($\Delta E_3$) or difference in brightness ($\Delta L_3$) of the hair sample located below the part with the UV-blocking ingredient applied before and after UV irradiation with the difference in chromaticity ($\Delta E_4$) or difference in brightness ($\Delta L_4$) of the hair sample below the part with the UV-blocking ingredient not applied before and after UV irradiation,
   wherein the UV-permeable plate is PMMA (polymethyl methacrylate) or quartz plate.

2. The method for evaluating the efficacy of a UV-blocking ingredient according to claim 1, wherein the fluorescent dye is dansyl chloride.

3. The method for evaluating the efficacy of a UV-blocking ingredient according to claim 2, wherein the hair sample is immersed in an organic solvent in which the dansyl chloride is dissolved for 0.5-48 hours.

4. The method for evaluating the efficacy of a UV-blocking ingredient according to claim 1, wherein the UV is irradiated with an intensity of 1,000-100,000 mJ/cm$^2$.

5. The method for evaluating the efficacy of a UV-blocking ingredient according to claim 1, wherein the chromaticity is a value measured according to CIE LAB.

6. The method for evaluating the efficacy of a UV-blocking ingredient according to claim 1, wherein the difference in chromaticity ($\Delta E_3$) of the hair sample located below the part with the UV-blocking ingredient applied before and after UV irradiation is calculated according to Equation 7, and the difference in chromaticity ($\Delta E_4$) of the hair sample below the part with the UV-blocking ingredient not applied is calculated according to Equation 8:

[Equation 7]

$$\Delta E_3 = ((L_6^* - L_5^*)^2 + (a_6^* - a_5^*)^2 + (b_6^* - b_5^*)^2)^{1/2}$$

wherein

$L_6^*$ is the brightness of the hair sample located below the part with the UV-blocking ingredient applied after UV irradiation, and $L_5^*$ is the brightness of the hair sample located below the part with the UV-blocking ingredient applied before UV irradiation,
$a_6^*$ is the chroma of the hair sample located below the part with the UV-blocking ingredient applied after UV irradiation, and $a_5^*$ is the chroma of the hair sample located below the part with the UV-blocking ingredient applied before UV irradiation, and
$b_6^*$ is the yellowness of the hair sample located below the part with the UV-blocking ingredient applied after UV irradiation, and $b_5^*$ is the yellowness of the hair sample located below the part with the UV-blocking ingredient applied before UV irradiation

[Equation 8]

$$\Delta E_4 = ((L_8^* - L_7^*)^2 + (a_8^* - a_7^*)^2 + (b_8^* - b_7^*)^2)^{1/2}$$

wherein

$L_8^*$ is the brightness of the hair sample located below the part with the UV-blocking ingredient not applied after

UV irradiation, and $L_7^*$ is the brightness of the hair sample located below the part with the UV-blocking ingredient not applied before UV irradiation,

$a_8^*$ is the chroma of the hair sample located below the part with the UV-blocking ingredient not applied after UV irradiation, and $a_7^*$ is the chroma of the hair sample located below the part with the UV-blocking ingredient not applied before UV irradiation, and

$b_8^*$ is the yellowness of the hair sample located below the part with the UV-blocking ingredient not applied after UV irradiation, and $b_7^*$ is the yellowness of the hair sample located below the part with the UV-blocking ingredient not applied before UV irradiation.

7. The method for evaluating the efficacy of a UV-blocking ingredient according to claim 6, wherein the UV cut-off rate is calculated according to Equation 9:

[Equation 9]

$$\text{UV cut-off rate (\%)} = ((\Delta E_4 - \Delta E_3) / \Delta E_4) \times 100.$$

8. The method for evaluating the efficacy of a UV-blocking ingredient according to claim 1, wherein the difference in brightness ($\Delta L_3$) of the hair sample located below the part with the UV-blocking ingredient applied before and after UV irradiation is calculated according to Equation 10, and the difference in brightness ($\Delta L_4$) of the hair sample located below the part with the UV-blocking ingredient not applied is calculated according to Equation 11:

[Equation 10]

$$\Delta L_3 = | L_6^* - L_5^* |$$

[Equation 11]

$$\Delta L_4 = | L_8^* - L_7^* |.$$

9. The method for evaluating the efficacy of a UV-blocking ingredient according to claim 8, wherein the UV cut-off rate is calculated according to Equation 12:

[Equation 12]

$$\text{UV cut-off rate (\%)} = ((\Delta L_4 - \Delta L_3) / \Delta L_4) \times 100.$$

**Patentansprüche**

1. Verfahren zum Bewerten der Wirksamkeit eines UV-blockierenden Wirkstoffs, umfassend:

einen Schritt eines Behandelns einer Haarprobe mit einem fluoreszierenden Farbstoff;
einen Schritt eines Messens einer Farbigkeit durch eine Fluoreszenzbildgebung der mit dem fluoreszierenden Farbstoff behandelten Haarprobe;
einen Schritt eines Aufbringens eines UV-blockierenden Wirkstoffs auf einen Teil einer UV-durchlässigen Platte;
einen Schritt eines Positionierens der mit dem fluoreszierenden Farbstoff behandelten Haarprobe unterhalb der UV-durchlässigen Platte;
einen Schritt eines Ausstrahlens von UV-Licht auf die Haarproben durch ein Ausstrahlen von UV-Licht von oberhalb der UV-durchlässigen Platte;
einen Schritt eines Messens einer Farbigkeit durch eine Fluoreszenzbildgebung der Haarproben; und
einen Schritt eines Berechnens einer UV-Cut-Off-Rate durch ein Vergleichen der Differenz einer Farbigkeit ($\Delta E_3$) oder der Differenz einer Helligkeit ($\Delta L_3$) der unterhalb des Teils mit dem vor und nach einer UV-Bestrahlung aufgebrachten UV-blockierenden Wirkstoff positionierten Haarprobe mit der Differenz einer Farbigkeit ($\Delta E_4$) oder

der Differenz einer Helligkeit ($\Delta L_4$) der Haarprobe unterhalb des Teils mit dem vor und nach einer UV-Bestrahlung nicht aufgebrachten UV-blockierenden Wirkstoff,
wobei die UV-durchlässige Platte eine PMMA- (Polymethylmethacrylat) oder eine Quarzplatte ist.

2. Verfahren zum Bewerten der Wirksamkeit eines UV-blockierenden Wirkstoffs nach Anspruch 1, wobei der fluoreszierende Farbstoff Dansylchlorid ist.

3. Verfahren zum Bewerten der Wirksamkeit eines UV-blockierenden Wirkstoffs nach Anspruch 2, wobei die Haarprobe für 0,5 - 48 Stunden in eine organische Lösung getaucht ist, in welcher das Dansylchlorid gelöst ist.

4. Verfahren zum Bewerten der Wirksamkeit eines UV-blockierenden Wirkstoffs nach Anspruch 1, wobei das UV-Licht mit einer Intensität von 1.000 - 100.000 mJ/cm$^2$ ausgestrahlt wird.

5. Verfahren zum Bewerten der Wirksamkeit eines UV-blockierenden Wirkstoffs nach Anspruch 1, wobei die Farbigkeit ein gemäß CIE LAB gemessener Wert ist.

6. Verfahren zum Bewerten der Wirksamkeit eines UV-blockierenden Wirkstoffs nach Anspruch 1, wobei die Differenz einer Farbigkeit ($\Delta E_3$) der unterhalb des Teils mit dem vor und nach einer UV-Bestrahlung aufgebrachten UV-blockierenden Wirkstoff positionierten Haarprobe gemäß Gleichung 7 berechnet wird und die Differenz einer Farbigkeit ($\Delta E_4$) der Haarprobe unterhalb des Teils mit dem nicht aufgebrachten UV-blockierenden Wirkstoff gemäß Gleichung 8 berechnet wird:

[Gleichung 7]

$$\Delta E_3 = ((L_6{}^* - L_5{}^*)^2 + (a_6{}^* - a_5{}^*)^2 + (b_6{}^* - b_5{}^*)^2)^{1/2}$$

wobei

$L_6{}^*$ die Helligkeit der unterhalb des Teils mit dem nach einer UV-Bestrahlung aufgebrachten UV-blockierenden Wirkstoff positionierten Haarprobe ist und $L_5{}^*$ die Helligkeit der unterhalb des Teils mit dem vor einer UV-Bestrahlung aufgebrachten UV-blockierenden Wirkstoff positionierten Haarprobe ist,
$a_6{}^*$ die Farbsättigung der unterhalb des Teils mit dem nach einer UV-Bestrahlung aufgebrachten UV-blockierenden Wirkstoff positionierten Haarprobe ist und $a_5{}^*$ die Farbsättigung der unterhalb des Teils mit dem vor einer UV-Bestrahlung aufgebrachten UV-blockierenden Wirkstoff positionierten Haarprobe ist und
$b_6{}^*$ die Gelbfärbung der unterhalb des Teils mit dem nach einer UV-Bestrahlung aufgebrachten UV-blockierenden Wirkstoff positionierten Haarprobe ist und $b_5{}^*$ die Gelbfärbung der unterhalb des Teils mit dem vor einer UV-Bestrahlung aufgebrachten UV-blockierenden Wirkstoff positionierten Haarprobe ist

[Gleichung 8]

$$\Delta E_4 = ((L_8{}^* - L_7{}^*)^2 + (a_8{}^* - a_7{}^*)^2 + (b_8{}^* - b_7{}^*)^2)^{1/2}$$

wobei

$L_8{}^*$ die Helligkeit der unterhalb des Teils mit dem nach einer UV-Bestrahlung nicht aufgebrachten UV-blockierenden Wirkstoff positionierten Haarprobe ist und $L_7{}^*$ die Helligkeit der unterhalb des Teils mit dem vor einer UV-Bestrahlung nicht aufgebrachten UV-blockierenden Wirkstoff positionierten Haarprobe ist,
$a_8{}^*$ die Farbsättigung der unterhalb des Teils mit dem nach einer UV-Bestrahlung nicht aufgebrachten UV-blockierenden Wirkstoff positionierten Haarprobe ist und $a_7{}^*$ die Farbsättigung der unterhalb des Teils mit dem vor einer UV-Bestrahlung nicht aufgebrachten UV-blockierenden Wirkstoff positionierten Haarprobe ist und
$b_8{}^*$ die Gelbfärbung der unterhalb des Teils mit dem nach einer UV-Bestrahlung nicht aufgebrachten UV-blockierenden Wirkstoff positionierten Haarprobe ist und $b_7{}^*$ die Gelbfärbung der unterhalb des Teils mit dem vor einer UV-Bestrahlung nicht aufgebrachten UV-blockierenden Wirkstoff positionierten Haarprobe ist.

7. Verfahren zum Bewerten der Wirksamkeit eines UV-blockierenden Wirkstoffs nach Anspruch 6, wobei die UV-Cut-Off-Rate gemäß Gleichung 9 berechnet wird:

[Gleichung 9]

UV-Cut-Off-Rate (%) = (($\Delta E_4 - \Delta E_3$) / $\Delta E_4$) × 100.

8. Verfahren zum Bewerten der Wirksamkeit eines UV-blockierenden Wirkstoffs nach Anspruch 1, wobei die Differenz einer Helligkeit ($\Delta L_3$) der unterhalb des Teils mit dem vor und nach einer UV-Bestrahlung aufgebrachten UV-blockierenden Wirkstoff positionierten Haarprobe gemäß Gleichung 10 berechnet wird und die Differenz einer Helligkeit ($\Delta L_4$) der unterhalb des Teils mit dem nicht aufgebrachten UV-blockierenden Wirkstoff positionierten Haarprobe gemäß Gleichung 11 berechnet wird:

[Gleichung 10]

$\Delta L_3 = | L_6^* - L_5^* |$

[Gleichung 11]

$\Delta L_4 = | L_8^* - L_7^* |$.

9. Verfahren zum Bewerten der Wirksamkeit eines UV-blockierenden Wirkstoffs nach Anspruch 8, wobei die UV-Cut-Off-Rate gemäß Gleichung 12 berechnet wird:

[Gleichung 12]

UV-Cut-Off-Rate (%) = (($\Delta L_4 - \Delta L_3$) / $\Delta L_4$) × 100.

**Revendications**

1. Procédé d'évaluation de l'efficacité d'un ingrédient bloquant les UV, comprenant :

une étape de traitement d'un échantillon de cheveux avec une teinture fluorescente ;
une étape de mesure de chromaticité par imagerie par fluorescence de l'échantillon de cheveux traité avec la teinture fluorescente ;
une étape d'application d'un ingrédient bloquant les UV sur une partie d'une plaque perméable aux UV ;
une étape de localisation de l'échantillon de cheveux traité avec la teinture fluorescente sous la plaque perméable aux UV ;
une étape d'irradiation d'UV sur les échantillons de cheveux par irradiation d'UV depuis le dessus de la plaque perméable aux UV ;
une étape de mesure de chromaticité par imagerie par fluorescence des échantillons de cheveux ; et
une étape de calcul d'un taux de coupure des UV en comparant la différence de chromaticité ($\Delta E_3$) ou la différence de luminosité ( $\Delta L_3$) de l'échantillon de cheveux situé sous la partie avec l'ingrédient bloquant les UV appliqué, avant et après l'irradiation UV, avec la différence de chromaticité ($\Delta E_4$) ou la différence de luminosité ($\Delta L_4$) de l'échantillon de cheveux situé sous la partie avec l'ingrédient bloquant les UV non appliqué, avant et après l'irradiation UV,
dans lequel la plaque perméable aux UV est une plaque de PMMA (polyméthacrylate de méthyle) ou de quartz.

2. Procédé d'évaluation de l'efficacité d'un ingrédient bloquant les UV selon la revendication 1, dans lequel la teinture fluorescente est le chlorure de dansyle.

3. Procédé d'évaluation de l'efficacité d'un ingrédient bloquant les UV selon la revendication 2, dans lequel l'échantillon de cheveux est immergé dans un solvant organique dans lequel le chlorure de dansyle est dissous pendant 0,5 à 48 heures.

4. Procédé d'évaluation de l'efficacité d'un ingrédient bloquant les UV selon la revendication 1, dans lequel les UV sont irradiés avec une intensité de 1 000 à 100 000 mJ/cm$^2$.

5. Procédé d'évaluation de l'efficacité d'un ingrédient bloquant les UV selon la revendication 1, dans lequel la chromaticité est une valeur mesurée selon CIE LAB.

6. Procédé d'évaluation de l'efficacité d'un ingrédient bloquant les UV selon la revendication 1, dans lequel la différence de chromaticité ($\Delta E_3$) de l'échantillon de cheveux situé sous la partie avec l'ingrédient bloquant les UV appliqué avant et après l'irradiation UV est calculée selon l'équation 7, et la différence de chromaticité ($\Delta E_4$) de l'échantillon de cheveux situé sous la partie avec l'ingrédient bloquant les UV non appliqué est calculée selon l'équation 8 :

[Équation 7]

$$\Delta E_3 = ((L_6^* - L_5^*)^2 + (a_6^* - a_5^*)^2 + (b_6^* - b_5^*)^2)^{1/2}$$

dans laquelle

$L_6^*$ est la luminosité de l'échantillon de cheveux situé sous la partie avec l'ingrédient bloquant les UV appliqué après l'irradiation UV, et $L_5^*$ est la luminosité de l'échantillon de cheveux situé sous la partie avec l'ingrédient bloquant les UV appliqué avant l'irradiation UV,
$a_6^*$ est la chromaticité de l'échantillon de cheveux situé sous la partie avec l'ingrédient bloquant les UV appliqué après l'irradiation UV, et $a_5^*$ est la chromaticité de l'échantillon de cheveux situé sous la partie avec l'ingrédient bloquant les UV appliqué avant l'irradiation UV, et
$b_6^*$ est le jaunissement de l'échantillon de cheveux situé sous la partie avec l'ingrédient bloquant les UV appliqué après l'irradiation UV, et $b_5^*$ est le jaunissement de l'échantillon de cheveux situé sous la partie avec l'ingrédient bloquant les UV appliqué avant l'irradiation UV

[Équation 8]

$$\Delta E_4 = ((L_8^* - L_7^*)^2 + (a_8^* - a_7^*)^2 + (b_8^* - b_7^*)^2)^{1/2}$$

dans laquelle

$L_8^*$ est la luminosité de l'échantillon de cheveux situé sous la partie avec l'ingrédient bloquant les UV non appliqué après l'irradiation UV, et $L_7^*$ est la luminosité de l'échantillon de cheveux situé sous la partie avec l'ingrédient bloquant les UV non appliqué avant l'irradiation UV,
$a_8^*$ est la chromaticité de l'échantillon de cheveux situé sous la partie avec l'ingrédient bloquant les UV non appliqué après l'irradiation UV, et $a_7^*$ est la chromaticité de l'échantillon de cheveux situé sous la partie avec l'ingrédient bloquant les UV non appliqué avant l'irradiation UV, et
$b_8^*$ est le jaunissement de l'échantillon de cheveux situé sous la partie avec l'ingrédient bloquant les UV non appliqué après l'irradiation UV, et $b_7^*$ est le jaunissement de l'échantillon de cheveux situé sous la partie avec l'ingrédient bloquant les UV non appliqué avant l'irradiation UV.

7. Procédé d'évaluation de l'efficacité d'un ingrédient bloquant les UV selon la revendication 6, dans lequel le taux de coupure des UV est calculé selon l'équation 9 :

[Équation 9]

$$\text{Taux de coupure des UV } (\%) = ((\Delta E_4 - \Delta E_3) / \Delta E_4) \times 100.$$

8. Procédé d'évaluation de l'efficacité d'un ingrédient bloquant les UV selon la revendication 1, dans lequel la différence de luminosité ($\Delta L_3$) de l'échantillon de cheveux situé sous la partie avec l'ingrédient bloquant les UV appliqué avant et après l'irradiation UV est calculée selon l'équation 10, et la différence de luminosité ($\Delta L_4$) de l'échantillon de cheveux situé sous la partie avec l'ingrédient bloquant les UV non appliqué est calculée selon l'équation 11 :

[Équation 10]

$$\Delta L_3 = | L_6^* - L_5^* |$$

[Équation 11]

$$\Delta L_4 = \mid L_8^* - L_7^* \mid.$$

9.  Procédé d'évaluation de l'efficacité d'un ingrédient bloquant les UV selon la revendication 8, dans lequel le taux de coupure des UV est calculé selon l'équation 12 :

[Equation 12]

$$\text{Taux de coupure UV (\%)} = ((\Delta L_4 - \Delta L_3) / \Delta L_4) \times 100.$$

【FIG. 1a】

【FIG. 1b】

【FIG. 2】

Before irradiation | After irradiation

【FIG. 3】

Before irradiation | After irradiation

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020200056015
- KR 20060127405
- JP H08271515 A
- US 2019285546 A1

**Non-patent literature cited in the description**

- Testing polysilicone-19 for hair conditioning and uv protection claims. *Cosmetic&Toiletries magazine*, 01 May 2008, vol. 123 (5)
- A sensitive fluorescence technique using dansyl chloride to assess hair damage. *Journal of the society cosmetic chemists, society of cosmetic chemists*, 01 September 1989, vol. 40 (5)